Europäisches Patentamt

(19) European Patent Office     (11) Numéro de publication: **0 229 578**
**B1**
Office européen des brevets

(12)                    **FASCICULE DE BREVET EUROPEEN**

(45) Date de publication du fascicule du brevet:     (51) Int. Cl.⁵: **A61F 2/36**
**10.10.90**

(21) Numéro de dépôt: **86420306.2**

(22) Date de dépôt: **18.12.86**

(54) Prothèse médicale autobloquante et procédés pour la fabriquer.

(30) Priorité: **24.12.85 FR 8519365**

(73) Titulaire: **Mai, Christian, 74 boulevard des Belges, F-69006 Lyon(FR)**
Titulaire: **Crassas, Yves, Clinique Saint Charles, F-38150 Roussillon(FR)**

(43) Date de publication de la demande: **22.07.87 Bulletin 87/30**

(45) Mention de la délivrance du brevet: **10.10.90 Bulletin 90/41**

(72) Inventeur: **Mai, Christian, 74 boulevard des Belges, F-69006 Lyon(FR)**
Inventeur: **Crassas, Yves, Clinique Saint Charles, F-38150 Roussillon(FR)**

(84) Etats contractants désignés:
**BE CH DE ES FR GB IT LI**

(74) Mandataire: **Laurent, Michel et al, Cabinet LAURENT et GUERRE B.P. 32, F-69131 Ecully Cédex(FR)**

(56) Documents cités:
**EP-A- 0 140 790**
**FR-A- 2 306 782**
**FR-A- 2 483 218**
**GB-A- 2 070 939**

**ARCH. ORT. E REUM., Vol. 94 fascicolo 1 (1981) "Materiale a memoria di forma: possibili utilizzi in campo ortopedico" par Airoldi et Parrini**

## Description

L'invention concerne une prothèse médicale autobloquante ; elle se rapporte également à un procédé pour la fabrication d'une telle prothèse.

Comme on le sait, une prothèse comprend essentiellement une tige destinée à être insérée dans un os soit pour le consolider, soit pour servir d'appui pour confectionner une articulation à remplacer.

Dans la suite de la description et dans les revendications, on se référera plus particulièrement à une prothèse de hanche étant entendu que cet exemple de réalisation n'est nullement limitatif, l'invention pouvant trouver d'autres applications, soit pour d'autres types d'articulation, soit pour, comme déjà dit, la consolidation d'un os quelconque.

Comme on le sait, une prothèse de hanche comprend essentiellement trois parties distinctes, à savoir respectivement :
– une tige fémorale destinée à être insérée dans le fémur ;
– une cupule destinée à être engagée dans le cotyloïde de la hanche à renforcer ;
– un col prothétique, coiffé d'une sphère, reliant la tige et la cupule, destiné à assurer l'articulation proprement dite.

A ce jour, on a déjà proposé des tiges de nature, de formes longitudinales ou de sections différentes. Le problème est de pouvoir fixer de façon efficace cette tige dans l'os (fémur).

On a ainsi proposé de fileter la tige , puis d'engager celle-ci dans un trou préparé dans l'os à l'instar d'une vis. Cette technique, bien que largement répandue, présente l'inconvénient de visser dans un os mou, ce qui entraîne à la longue un certain jeu , et affecte la stabilité de la prothèse.

On a également proposé de fixer alors la tige dans l'os au moyen d'un ciment, notamment à base de polymère acrylique, tel que du polyméthacrylate de méthyle. Ce ciment vieillit mal dans le temps, ainsi que sous l'effet des contraintes dues au mouvement et au poids du corps. Cela a pour effet diminuer les caractéristiques mécaniques du ciment et donc d'entraîner un certain risque de descellement de la prothèse. En outre, lors de la réaction de polymérisation du ciment, la température du ciment atteint facilement 70°C, température relativement importante qui peut provoquer des nécroses du tissu osseux.Enfin, si pour des raisons diverses, il devient nécessaire de procéder au changement de cette prothèse, l'opération devient problématique, pour ne pas dire impossible.

Dans les brevets FR-A-2 483 218 et EP-A-0050533, on a proposé de ménager une fente longitudinale dans la tige pour donner une certaine élasticité à cette pointe et ainsi favoriser l'engagement de la tige dans l'os qui doit néanmoins toujours être effectué de force pour bien assurer le blocage de la tige dans le canal médulaire. Mais ce blocage crée un risque d'arrachement du tissu spongieux sous l'effet du temps et des mouvements de la hanche, donc un risque de descellement. En outre, cette solution, comme toutes les solutions connues à ce jour, à l'exception de la solution dans laquelle la tige est filetée, présente une difficulté sinon une impossibilité d'être retirée ultérieurement, ce qui rend ainsi impossible la mise en place de prothèse sur des enfants ou sur des adolescents.

Dans le document US-A 4 170 990, on a proposé de réaliser des broches ou des agraphes médicales avec des alliages à base de titane présentant un effet de mémoire de forme. On peut ainsi, en jouant avec la température, mettre en place et retirer ces éléments une fois la blessure réparée.

Dans la publication de Monsieur G. AIROLDI et L. PARRINI (ARCH.ORT.E REUM – vol. 94, fasc. 1, 1981, pages 17 à 35), on a suggéré (voir notamment figure 20a et page 31) de réaliser des tiges de prothèse sous forme d'un tube fendu longitudinalement selon une génératrice en un matériau à mémoire martensitique. De la sorte, après insertion de cette tige dans le canal médulaire de l'os à renforcer, le tube s'expanse sous l'effet d'une élévation de température. On obtient ainsi un serrage uniforme tout le long du canal médulaire qui malheureusement, ne s'oppose pas à la rotation de la tige une fois en place. En outre, l'épaisseur des tubes est jugée encore trop insuffisante pour assurer une bonne rigidité à l'ensemble.

L'invention pallie ces inconvénients. Elle vise une tige de prothèse du type en question, autobloquante, en un matériau martensitique, facile à insérer et qui assure non seulement un blocage progressif de la tige dans le canal médulaire de l'os à renforcer, mais qui également s'oppose aux rotations de la tige une fois insérée.

Cette tige de prothèse autobloquante, destinée à être insérée dans un os, fendue sur partie de sa longueur de manière à présenter une section transversale deformable, et réalisée en un matériau biologiquement compatible, qui présente une mémoire de forme et une température de transformation martensitique Ms inférieure à la température du corps humain, se caractérise en ce que cette tige comporte trois portions distinctes, à savoir respectivement:

– tout d'abord, une première portion terminale, disposée à 1, extrémité de la tige, et qui présente une fente longitudinale de manière à former au moins deux branches distinctes;
– puis une deuxième portion médiane pleine;
– enfin, une troisième portion, disposée au voisinage du raccord de la tige avec la tête, qui comprend des fentes orthogonales à la fente longitudinale de la première portion, et qui s'étendent sur partie de la hauteur de cette troisième portion.

Avantageusement, en pratique:

– la tête de la tige, opposée à la pointe, présente un alésage destiné à recevoir un organe chauffant lorsque l'on désire écarter les pointes des branches, soit un organe refroidissant lorsque, au contraire, on désire rapprocher ces branches;
– la tige est réalisée en une seule pièce et la troisième portion comprend deux fentes parallèles usinées dans la masse de la troisième portion, et qui s'étendent sur pratiquement toute la hauteur de cette troisième portion;

– le matériau de la tige est un alliage de titane ayant une température de transformation martensitique Ms comprise entre 0 et 37°C, de préférence de l'ordre de 0 à 20°C, avantageusement au voisinage de 15°C.

En pratique, ces fentes parallèles sont obtenues par usinage dans la masse de la troisième portion.

De la sorte, cette disposition permet d'assurer une excellente répartition du serrage tout le long du canal médulaire de l'os et par là, une meilleure prise et ce, sans fragiliser tige par une fente qui irait de la pointe à la tête.

Le phénomène de "mémoire de forme" est dû à la transformation martensitique thermoélastique réversible. Ce phénomène est bien connu. Il n'y a donc pas lieu de le décrire ici en détail. Ce phénomène consiste à donner à un matériau une forme que l'on traite à une température T1 supérieure à la température de transformation martensitique Ms puis à lui donner une autre forme à une température T2 inférieure à cette température Ms, et enfin à répéter plusieurs fois cette opération en fonction de la nature de l'alliage utilisé, afin de donner à ce matériau sa mémoire définitive. L'invention consiste à mettre en oeuvre ce phénomène bien connu depuis fort longtemps dans le domaine spécifique des prothèses médicales.

Les matériaux mis en oeuvre doivent bien évidemment être compatibles biologiquement avec le milieu auxquels ils sont destinés. En outre, comme déjà dit, ces matériaux doivent présenter une mémoire de forme et une température de transformation martensitique Ms inférieure à la température du corps humain. Cette température Ms dépend de la concentration des constituants de l'alliage et se trouve accessible dans la littérature. Comme matériaux susceptibles d'être mis en oeuvre, on peut citer des alliages à base detitane-nickel, de cuivre-manganèse, de cuivre-zinc, de platine-fer, de cuivre-aluminium-zinc. Dans une forme avantageuse, on peut revêtir la prothèse d'une couche protectrice, notamment en un matériau biocompatible, tel que le titane, déposé par tout moyen connu, voire d'une couche de céramique, sous réserve bien évidemment que cette couche de protection soit compatible biologiquement avec le milieu osseux auquel elle est destinée.

Le choix du matériau destiné à réaliser la tige est essentiellement dicté par des considérations de prix de revient, de résistnace mécanique, d'inertie biologique et de conditions d'usinage.

Comme déjà dit,la tige comporte dans sa partie inférieure de deux à plusieurs branches que l'on peut réaliser par toute technique connue, telle que moulage, forgeage, frittage usinage. On a constaté qu'au-delà de quatre branches, on augmentait inutilement le coût de la prothèse sans amélioration proportionnelle, avec en outre un risque de fragilisation pour des petites prothèses.

La manière dont l'invention peut être réalisée et les avantages qui en découlent ressortiront mieux des exemples de réalisation qui suivent donnés à titre indicatif et non limitatif, à l'appui des figures annexées.

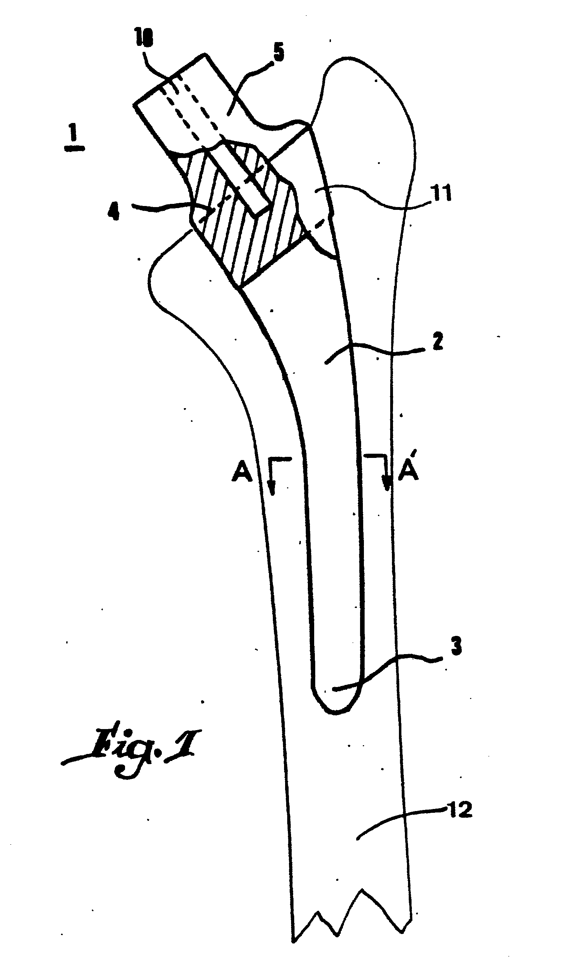

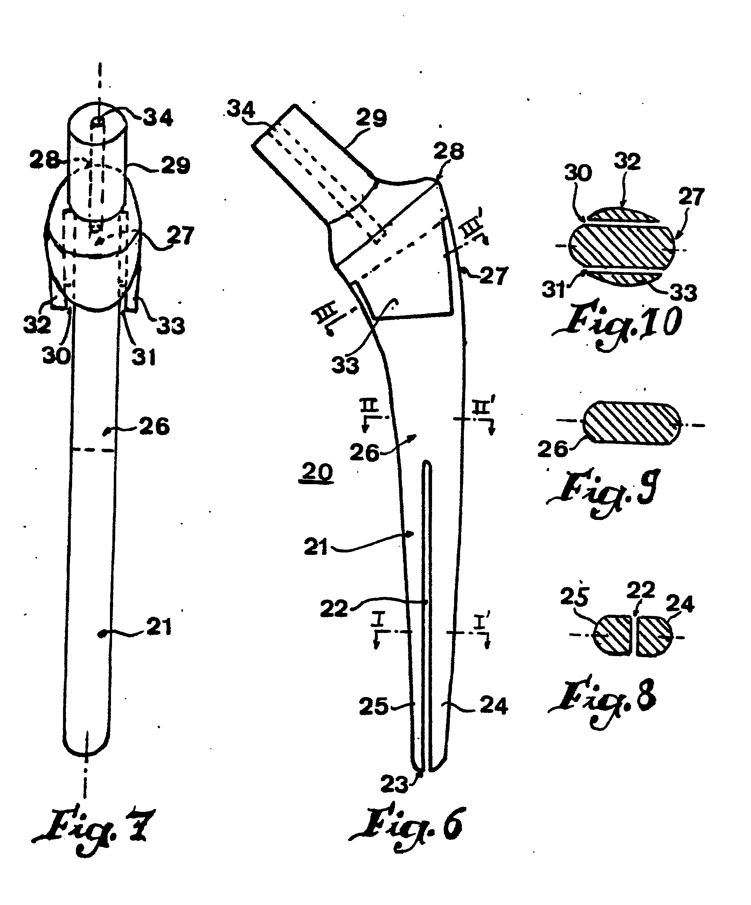

Il est entendu que la tige fémorale représentée aux figures 1 à 5 ne fait pas partie de l'objet de l'invention, la quelle demeure restreinte à la forme de réalisation exempli fiée aux figures 6 à 10 et telle que definie dans la revendication indépendante. La description relative aux figures 1 à 5 est toutefois inelue pour une meilleure compréhension de l'invention.

La figure 1 est une représentationschématique en coupe longitudinale d'une tige fémorale de prothèse de hache dans une forme de réalisation en soi connue.

Les figures 2 et 3 représentent une telle tige fémorale vue respectivement de devant en position branches écartées (figure 2) et en position branches repliées (figure 3).

Les figures 4 et 5 montrent une coupe selon l'axe AA' de la figure 1 respectivement en position branches écartées (figures 2 et 5) et en position branches repliées (figures 3 et 4), mais à plus grande échelle pour en faciliter la compréhension.

Les figures 6 à 10 montrent schématiquement une forme de réalisation préférée de l'invention, représentée respectivement en vue longitudinale (figure 6), vue de devant (figure 7) et vue en coupe selon I-I' (fig. 8), selon II-II' (fig. 9) et selon III-III' (fig. 10.).

La tige fémorale désignée par la référence générale (1) comprend essentiellement une tige proprement dite (2) effilée vers sa pointe (3) comportant sur la face opposée un renflement (4) et un col prothétique (5) destiné à recevoir la sphère d'articulation.

Par usinage, on taille dans la tige (2) une fente (6) d'environ 1, 5 millimètre sur approximativement la moitié de la longueur de la tige (2). Cette fente est effectuée dans le sens de la courbure de la tige (1) mais elle peut l'être également dans un autre sens. Cette fente définit ainsi deux branches (7) et (8) distinctes.

Le matériau constituant la tige (1) est un alliage titane-nickel (49,3 % atomique de titane et 50,7 % de nickel) dont la température de transformation martensitique Ms est voisine de 15°C.

Mécaniquement, à température ambiante, on introduit un coin cans l'espace (9) défini à l'extrémité (3), de manière à écarter les deux branches (7) et (8) en portant la distance au niveau de l'extrémité (3) à environ six millimètres (figure 5). Cette opération étant effectuée à une température T1 supérieure à Ms (15°C), la tige acquiert une première mémoire de cette forme écartée.

On retire le coin de la pointe (9) puis on trempe la tige dans de l'eau glacée tout en resserrant l'extrémité (3) des branches (7) et (8) écartées, par exemple au moyen d'un collier de force. La tige acquiert ainsi cette mémoire de la forme repliée puisque cette température T2 de 0°Cest inférieure à la température Ms.

On répète dix fois de suite ces opérations T1 et T2

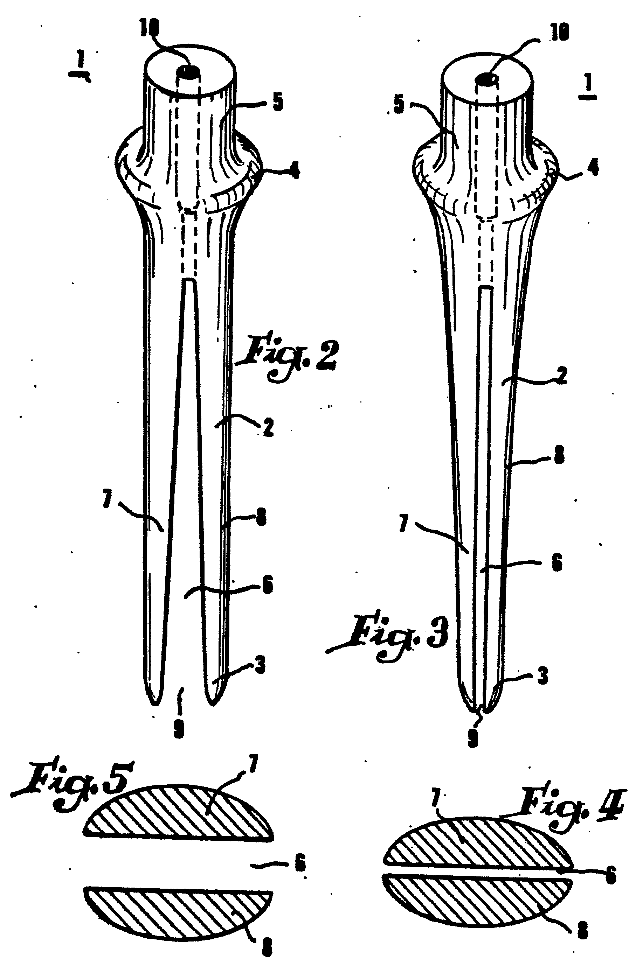

On obtient ainsi une tige fémorale (1) ayant la mémoire des formes écartée (T1) et repliée (T2), mais qui, à température ambiante, se présente donc sous forme de branches élémentaires (7,8) écartées (figure 5). Avantageusement, la section périphérique de la pointe (figure 5) en position écartée est sensiblement voisine de la section périphérique de la portion de la tige non fendue (figure 2) pour bien assurer la mise en place et le blocage dans le trou ménagé à cet effet dans l'os du fémur (12).

La tige fémorale (1) présente également un alésage (10) ménagé dans le col prothétique (5) et pénétrant de manière appréciable dans la partie pleine de la tête (11) de la tige (1). Avantageusement, cette partie (11) comporte de manière connue des alvéoles ou des microporosités destinées à faciliter la prolifération ultérieure des tissus osseux lors de la repousse. De même, ces microrugosités peuvent recouvrir toute la tige (1).

Lorsque le chirurgien désire mettre en place une tige prothétique selon l'invention, il prépare de manière connue un trou dans le fémur (12). Pour replier les extrémités des branches (7) et (8) écartées, il trempe la tige dans de l'eau glacée. Ainsi, les extrémités des branches sont repliées l'une vers l'autre par l'effet de mémoire de forme. Le chirurgien peut donc alors sans aucune difficulté introduire la tige (1) dans le trou préparé à cet effet. Il introduit alors dans l'alésage (10) un élément chauffant, tel qu'une résistance alimentée par une pile électrique. Lorsque la température de l'ensemble de la tige (1) excède 15°C, sous l'effet de la mémoire de forme, les branches (7) et (8) s'ecartent progressivement pour bien venir se plaquer et s'appuyer fortement contre les rebords du trou ménagé dans le canal médulaire de l'os (12) en s'écartant comme montré à la figure 5.

De la sorte, le blocage est parfaitement assuré.

Si pour une raison quelconque, le chirurgien, même en cours d'opération, désire retirer la prothèse, il suffit d'introduire dans l'alésage (10) un élément refroidissant. Dès que la température de la tige (1) descend en dessous de 15°C, sous l'effet de ce corps refroidissant les branches (7, 8) se replient. Il est alors possible de retirer sans aucun effort la tige, puis de la remettre en place ou s'il y a lieu de la changer.

Dans une autre forme de réalisation, il n'est pas nécessaire d'introduire un élément chauffant dans l'alésage (10), la montée en température étant obtenue progressivement par échange avec le corps humain.

Ainsi, tant que la température de la tige reste au dessus de la température Ms, la prothèse reste solidement bloquée de façon homogène. Pour désceller cette prothèse à un moment quelconque et pour une raison quelconque, il suffit de refroidir la tige à une température inférieure à Ms et ce, par un moyen quelconque. Ainsi, le descellement se fait en douceur et sans risque de traumatisme osseux.

Contrairement à l'art antérieur, la mise en place de la tige se fait en douceur et cette opération de mise en place peut se répéter plusieurs fois au cours même de l'intervention chirurgicale ou même ultérieurement. Ainsi, ce type de prothèse peut être même utilisé sur des enfants ou des adolescents, puisque, en fonction de la croissance, on peut retirer facilement la prothèse en question.

Dans la forme de réalisation préférée montrée aux figures 6 à 10, la tige (20) de cette prothèse présente une première portion (21) qui comporte une fente longitudinale unique (22) qui s'étend depuis la pointe (23) et sur la moitié environ de la longueur de la tige (20) afin de former deux branches distinctes (24,25) (fig. 6,8).

Cette portion fendue (21) se raccorde ensuite à une deuxième portion pleine (26) montrée à la figure 9.

A son tour, cette portion (26) se raccorde à une troisième portion (27) disposée au voisinage du raccord (28) de la tige (20) avec la tête (29) donc au niveau du trochanter. Selon une caractéristique de l'invention, cette troisième portion (27) comprend deux fentes parallèles (30,31) usinées, orthogonales à la fente (22) (figures 6-10) et qui s'étendent (figure 7) sur pratiquement toute la hauteur de cette troisième portion (27).

Ainsi, ces fentes (30,31) forment deux pans parallèles (32,33) disposées de part et d'autre de (27),mais monobloc abec (27). La référence (34) désigne un alésage analogue à (10).

L'ensemble de la prothèse (21,26,27,32,33) est donc monobloc et en un matériau martensitique.

On confère à l'ensemble la mémoire de forme comme précédemment.

Cette disposition assure une excellente stabilité en assurant un plaquage parfait des branches (24,25) contre le canal médulaire et des pans (32,33) au niveau du trochanter, ce qui empêche la rotaton de la tige (20).

Les prothèses de l'invention sont particulièrment adaptées aux hanches ou autres articulations.

**Revendications**

1. Prothése autobloquante, dont la tige (20) destinée à être insérée dans un os:
   - d'une part, est fendue (22) sur partie de sa longeur, de manière à présenter une section transversale déformable;
   - et d'autre part, est réalisée en un matériau biologiquement compatible, qui présente une mémoire de forme et une température de transformation martensitique inférieure à la température du corps humain, caractérisée en ce que cette tige comporte trois portions distinctes, à savoir respectivement:
   - tout d'abord, une première portion terminale (21), disposée à l'extrémité (23) de la tige (20), et qui présente une fente longitudinale (22) de manière à former au moins deux branches (24, 25) distinctes;
   - puis une deuxième portion médiane (26) pleine;
   - enfin, une troisième portion (27), disposée au voisinage du raccord (28) de la tige (20) avec la tête (29), qui comprend des fentes (30, 31) orthogonales à la fente (22) longitudinale de la première portion (21), et qui s'étendent sur partie de la hauteur de cette troisième portion (27).

2. Prothèse selon la revendication 1, caractérisée en ce que la tête (29) de la tige (20), opposée à la pointe (23) présente un alésage (34) destiné à recevoir un organe chauffant (pour écarter les branches (24-25)) ou refroidissant (pour rapprocher cesdites branches).

3. Prothése selon la revendication 1, du type dans laquelle la tige (20) est réalisée en une seule pièce, caractérisée en ce que la trosième portion (27) comprend deux fentes parallèles (30, 31) usinées dans la masse de cette troisième portion (27) et qui s'étendent sur pratiquement toute la hauter de cette troisième portion (27).

4. Prothèse selon la revendication 1, caractérisée en ce que le matériau de la tige (20) est un alliage de titane ayant une température de transformation martensitique Ms voisine de 15°C.

## Claims

1. Self-locking prothesis, in which the pin (20) entitled to be inserted into a bone:
   - at one hand, is slited (22) over part of its length, so as to present a deformable cross-section;
   - and at a second hand, is produced in a biologically compatible material having a sharp memory effect and having a martensite transformation temperature lower than the human body temperature; characterized in that this pin comprises three separate sections, namely:
   - at once, a first end portion (21) located at the end (23) of the pin (20) and which comprises a longitudinal slit (22), in order to form at least two separate branches (24, 25);
   - then, a second solid middle portion (26);
   - lastly, a third portion (27) situated close to the junction (28) of the pin (20) with the head (29) which comprises slits (30, 31) orthogonal to the longitudinal slit (22) of the first portion (21), and which extends over part of the height of said third portion (27).

2. Prothesis according to claim 1, characterized in that the head (29) of the pin (20), opposed to the end (23), comprises a bore (34) designed to receive a heating element (to spread out the branches (24, 25) or a cooling element (to draw up said branches).

3. Prothesis according to claim 1, in which the pin (20) is in a one piece material, characterized in that the third portion (27) comprises two parallel slits (30, 31) cut in the mass of said third portion and extending through nearly the entire height of said third portion (27).

4. Prothesis according to claim 1, characterized in that the material of the pin (20) is a titanium alloy having a martensite transformation temperature Ms in the proximity of 15°C.

## Patentansprüche

1. Selbsthemmende Prothese, deren Schaft (20), der in einen Knochen eingesetzt werden soll,
   - einerseits, über einen Teil seiner Länge derart geschlitzt (22) ist, daß er einen nachgiebigen Querschnittsbereich aufweist;
   - und andererseits, aus einem biologisch verträglichen Material hergestellt ist, das ein Formengedächtnis und eine martensitische Umwandlungstemperatur aufweist, die unterhalb der Temperatur des menschlichen Körpers liegt, dadurch gekennzeichnet, daß der Schaft drei verschiedene Abschnitte aufweist, nämlich,
   - zunächst, einen, am Ende (23) des Schaftes (20) gelegenen ersten Endabschnitt (21), der einen solchen längsverlaufenden Spalt (22) aufweist, daß zumindest zwei getrennte Schenkel (24, 25) gebildet sind;
   - ferner, einen zweiten mittleren massiven Abschnitt (26);
   - schließlich, einen dritten Abschnitt (27), der in der Nähe der Verbindungsstelle (28) des Schaftes (20) mit dem Kopf (29) angeordnet ist, und der Spalte (30, 31) aufweist, die orthogonal zum längsverlaufenden Spalt (22) des ersten Abschnittes (21) verlaufen, und die sich über einen Teil der Höhe des dritten Abschnittes (27) erstrecken.

2. Prothese nach Anspruch 1, dadurch gekennzeichnet, daß der Kopf (29) des Schaftes (20), gegenüberliegend zur Endstelle (23) eine Bohrung (34) aufweist, die dazu bestimmt ist, ein Heizorgan (zum Spreizen der Schenkel (24, 25)) oder ein Kühlorgan (zum Annähern dieser Schenkel) aufweist.

3. Prothese nach Anspruch 1, bei der der Schaft (20) aus einem einzigen Stück hergestellt ist, dadurch gekennzeichnet, daß der dritte Abschnitt (27) zwei parallele Spalte (30, 31) aufweist, die in die Masse des dritten Abschnittes (27) eingearbeitet sind, und die sich praktisch über die gesamte Höhe des dritten Abschnittes (27) erstrecken.

4. Prothese nach Anspruch 1, dadurch gekennzeichnet, daß das Material des Schaftes (20) eine Titanlegierung ist, die eine martensitische Umwandlungstemperatur Ms in der Nähe von 15°C aufweist.

*Fig. 1*

*Fig. 2*

*Fig. 3*

*Fig. 5*

*Fig. 4*

Fig. 7

Fig. 6

Fig. 10

Fig. 9

Fig. 8